# EUROPEAN PATENT SPECIFICATION

(11) **EP 0 355 983 B1**
(45) Date of publication and mention of the grant of the patent: **15.09.1993**
(21) Application number: 89307225.6
(22) Date of filing: 17.07.1989
(51) Int. Cl.: C07C 69/017, C07C 67/297, C07C 49/825, C07C 45/46, C07C 67/08, C07C 67/29, C08F 12/24

(54) **A process for the low residence time dehydration of p-acetoxyphenyl methylcarbinol**
Verfahren zur kurzzeitigen Dehydratation von Para-Acetoxyphenylcarbinol
Procédé de déshydratation avec un temps de séjour court du para-acétoxyphénylcarbinol

(30) Priority: 19.07.1988 US 221146
(43) Date of publication of application: 28.02.1990
(73) Proprietor: HOECHST CELANESE CORPORATION, Somerville, N.J. 08876 (US)
(72) Inventor: Shah, Bakulesh N., Corpus Christi Texas (US); Tran, Dung Q., Corpus Christi Texas (US); Keene, Donna L., Carrollton Virginia (US)
(74) Representative: De Minvielle-Devaux, Ian Benedict Peter

(56) References cited:
- US-A- 2 748 160
- THE JOURNAL OF ORGANIC CHEMISTRY, vol. 23, no. 4, 18th April 1958, pages 544-549, The American Chemical Society; B.B. CORSON et al.: "Preparation of vinylphenols and isopropenylphenols"

## Description

The present invention relates to a process for the low residence time dehydration of 4-acetoxyphenyl methylcarbinol in the production of 4-acetoxystyrene. The invention also pertains to a process for the polymerization, and hydrolysis of 4-acetoxystyrene. It is known in the art to produce monomers, homopolymers and copolymers of 4-acetoxystyrene and to hydrolyze the same to produce 4-hydroxystyrene derivatives or poly(4-hydroxystyrene). These compounds may be employed in the production of adhesives, coating compositions, photoresists and the like. Poly(4-hydroxystyrene) compounds are useful as binder resins for photoresists. Alpha acetoxystyrene and beta acetoxystyrene are known compounds and are described in U.S. Patent 4,144,063 and acetoxymethylstyrene is described in U.S. Patent 3,963,495. U.S. patent 4,075,237 describes 1,4-dimethyl-2-hydroxystyrene, while U.S. Patent 4,565,846 teaches the use of poly(3,5-dimethyl-4-hydroxystyrene). Japanese patent 84023747 describes anti-static compounds employing polyacetoxymethylstyrene and U.S. Patent 4,221,700 describes a stabilized synthetic polymer composition using poly(alkylated alkenylphenol) including 2-methyl paravinyl phenol. U.S. Patents 4,600,683 and 4,543,397 describe poly (alphamethyl vinylphenol). U.S. Patents 4,517,028; 4,460,770 and 4,539,051 describe dimethyl vinyl phenol.

The preparation of 4-hydroxystyrene is well known in the art. One process is described in U.S. Patent 4,316,995 and another is described in U.S. Patent 4,451,676. Vinyl phenol may be prepared by a five step process of (1) acetylating phenol to p-hydroxyacetophenone, (2) acetylation of p-hydroxyacetophenone to p-acetoxyacetophenone, (3) hydrogenation to p-acetoxyphenyl methylcarbinol, (4) dehydration to p-acetoxystyrene, and (5) saponification to p-vinylphenol. The method is more fully described in Corson, B. B., et al, Preparation of Vinylphenols and Isopropenylphenols, J. Org. Chem., April 1958.

Known processes for the hydrogenation of 4-acetoxyacetophenone to acetoxyphenyl methylcarbinol have been conducted with a Pd/C catalyst. This method has required the presence of such solvents as methanol and tetrahydrofuran. While this hydrogenation may be used in conjunction with the dehydration process of this invention, it is not the most preferred embodiment since the use of the solvent necessarily requires its ultimate removal. Problems which have been experienced include the dissolution of the 4-acetoxyacetophenone in the solvent; increased reactor volume due to the presence of the solvent; subsequent removal of the Pd/C catalyst from the large volume of solvent in the 4-acetoxyphenyl methylcarbinol mixture; separation of the solvent from 4-acetoxyphenyl methylcarbinol; purification and recycling of the solvent; solvent losses; and by-products from the solvent. The present invention preferably employs a process wherein the solvent is completely eliminated. By this means it has been found that the selectivity of the reaction is substantially not adversely affected by the elimination of the solvent component. Also, other side reactions are not observed and an economical operation of the process is made possible.

This invention more specifically provides an improved method for dehydrating 4-acetoxyphenyl methylcarbinol. This is performed by heating at 85^{o}C to 300^{o}C under a vacuum of 0.1 mm HgA to 760 mm HgA (1,013.10⁵ Pa to 13,3 Pa), preferably for about ten minutes or less and more preferably about two minutes or less in the presence of an acid catalyst. Conventional practice conducts this reaction in a batch reactor. Unfortunately, the batch process required about three hours. The present invention substantially improves reaction time by preferably employing a low residence time thin film evaporator, preferably in conjunction with a distillation column. As a result, a reaction selectivity increase of at least about 10% is observed.

### Summary of the Invention

The invention provides a method for producing 4-acetoxystyrene which comprises heating 4-acetoxyphenyl methylcarbinol, in the presence of a catalytic amount of an acid catalyst, at a temperature of from 85°C to 300°C under a pressure of from 0.1 mm HgA to 760 mm HgA (13,3 Pa to 1,013.10⁵ Pa) in a thin film evaporator for a period of time of from .2 minutes to 10 minutes.

The invention also provides a process for the production of 4-acetoxystyrene which comprises:
a) acylating phenol with acetic anhydride to produce 4-hydroxyacetophenone; and
b) acylating the 4-hydroxyacetophenone with acetic anhydride to form 4-acetoxyacetophenone; and
c) hydrogenating 4-acetoxyacetophenone to produce 4-acetoxyphenyl methylcarbinol. This is most preferably performed by heating 4-acetoxyacetophenone at a temperature of from 54°C to 120°C in the presence of at least a stoichiometric amount of hydrogen, and a catalytic amount of a catalyst selected from the group consisting of Pd/C or activated nickel such as Raney Nickel in the absence of a solvent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300°C under a pressure of from 0.1 mm HgA to 760 mm HgA (13,3 Pa to 1,013.10⁵ Pa) in a thin film evaporator for a period of time of from .2 minutes to 10 minutes.

The invention also provides a process for the production of poly (4-acetoxystyrene) which comprises subsequent free radical polymerization of the 4-acetoxystyrene to form poly(4-acetoxystyrene) having a molecular weight in the range of from 1,000 to 800,000, preferably 5,000 to 500,000.

The invention still further provides a process for the production of poly(4-hydroxystyrene) which comprises subsequently hydrolyzing the poly(4-acetoxystyrene) to form poly(4-hydroxystyrene) having a molecular weight in the range of from 1,000 to 500,000, preferably 5,000 to 500,000.

### Detailed Description of the Preferred Embodiment

In the process for the production of 4-acetoxystyrene, one begins with phenol and acylates it with acetic anhydride via a Friedel-Crafts catalysis or Fries rearrangement to produce 4-hydroxyacetophenone. This 4-hydroxyacetophenone is then esterified with acetic anhydride to form 4-acetoxyacetophenone. The latter is then hydrogenated, preferably neat hydrogenated or reduced to form 4-acetoxyphenyl methylcarbinol. This is then dehydrated with an acid or base to form 4-acetoxystyrene monomer. Free radical polymerization and hydrolysis may follow.
A typical overall reaction sequence may be described schematically as follows:

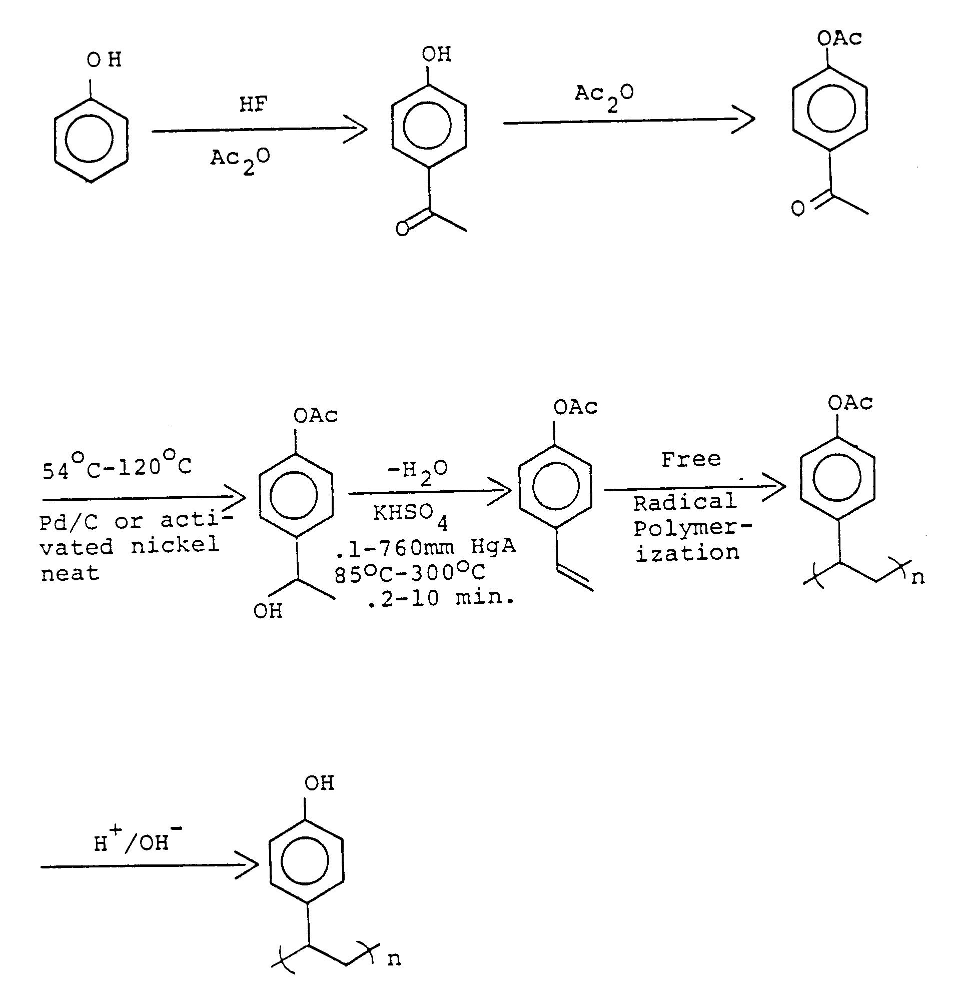

In the preferred embodiment the first reaction steps proceed as follows. One charges a reaction vessel with phenol and a slight excess of acetic anhydride and a Friedel-Crafts catalyst such as hydrogen fluoride. The acylation is conducted at a temperature of from 5^{o}C to 100^{o}C, or more preferably from 20^{o}C to 80^{o}C. A most preferred temperature is about 50^{o}C. The reaction proceeds at a preferred pressure of from 700 mm Hg to 780 mm Hg (0,465.10⁵ Pa to 1,037.10⁵ Pa) for from 1 to 5 hours. Although hydrogen fluoride is the preferred catalyst, other Lewis acids may be also used such as AlCl₃, BF₃, HClO₄, FeCl₃ and SnCl₄. In the alternative, the acylation may be conducted by a Fries rearrangement, in a manner well known to the skilled artisan. The reaction product is 4-hydroxyacetophenone. This 4-hydroxyacetophenone is then esterified with acetic anhydride. In this process, the 4-hydroxyacetophenone is refluxed with an excess of acetic anhydride for from 2 to 6 hours. Excess acetic anhydride as well as generated acetic acid are removed by distillation in vacuo. This is conducted, for example at a pressure of from .1 to 1,000 mm Hg (13,3 Pa to 1,33.10⁵ Pa) and at an initial temperature of from 135^{o}C to 150^{o}C, preferably from 135^{o}C to 145^{o}C which is then reduced to from 35^{o}C to 85^{o}C.

The 4-acetoxyacetophenone is then neat hydrogenated to 4-acetoxyphenyl methylcarbinol. This is performed by heating 4-acetoxyacetophenone at a temperature of from 54^{o}C to 120^{o}C in the presence of at least a stoichiometric amount of hydrogen, and a catalyst selected from the group consisting of Pd/C or activated nickel such as Raney Nickel in the absence of a solvent, for a sufficient time to produce acetoxyphenyl methylcarbinol. In the more preferred embodiment, the reaction is conducted at a temperature of from 60^{o}C to 90^{o}C. In the preferred embodiment, the reaction is conducted until substantial completion of hydrogenation as indicated by a lack of H₂ uptake. In the preferred embodiment, when Pd/C is used, the reaction proceeds at a pressure of from 14.7 psig to 5,000 psig (2,026.10⁵ Pa to 345,74.10⁵ Pa) more preferably at a pressure of from 50 psig to 500 psig (4,460.10⁵ Pa to 35,486.10⁵ Pa) and most preferably at a pressure of from 100 psig to 400 psig (7,907.10⁵ Pa to 28,591.10⁵ Pa). When activated nickel is used the reaction proceeds at a pressure of from 14.7 to 5,000 psig (2,026.10⁵ Pa to 345,74.10⁵ Pa), more preferably from 300 to 680 psig (21,693.10⁵ Pa to 47,893.10⁵ Pa) and most preferably from 350 to 450 psig (25,143.10⁵ to 32,033.10⁵ Pa). Activated nickel is the preferred catalyst since it can be recycled and a process with a higher selectivity is attained. In another embodiment, although lesser preferred, the 4-acetoxyacetophenone is hydrogenated with a suitable reagent to form the 4-acetoxyphenyl methylcarbinol. One preferred reagent is NaBH₄. Other reagents non-exclusively include lithium aluminum hydride, hydrogen, and diisobutyl aluminum hydride. The solvent may be ethanol, methanol or tetrahydrofuran. Other methods of hydrogenation DLK are discussed in Corson, above, at page 548, which is incorporated herein by reference.

This product is then dehydrated. Several prior known batch dehydration methods are taught by Corson. Dehydration is preferably conducted by vacuum heating in the presence of a polymerization inhibitor and a dehydrating agent. In one preferred embodiment, the 4-acetoxyphenyl methylcarbinol is mixed with a KHSO₄ dehydrating agent and t-butyl catechol as a polymerization inhibitor. Other useful dehydrating agents non-exclusively include alumina, titania, silica gel and mineral acids. Other polymerization inhibitors non-exclusively include hydroquinone, tetrachloroquinone tert-butyl catechol, phenothiazine, and di-t-butyl-p-cresol. The dehydrating agent is present in an amount of from 0.25 to 5.0 percent by weight of the 4-acetylphenyl methylcarbinol.

The polymerization inhibitor is preferably present in an amount of from 1% to 5% based on the weight of the 4-acetylphenyl methylcarbinol.

The reaction is conducted in a low residence time thin film evaporator, the output of which connects to a distillation column. The 4-acetoxyphenyl methylcarbinol, catalyst and polymerization inhibitor are metered into the evaporator or the distillation column at a rate sufficient to achieve the desired residence time. A thin film of the carbinol mixture is constantly wiped by a wiper rotating in the evaporator. The evaporator may be heated with hot oil or steam.

The reaction vessel is heated to from 85^{o}C to 300^{o}C, preferably 170^{o}C to 250^{o}C at a pressure of from 0.1 mm HgA to 760 mm Hg (13,3 Pa to 1,013.10⁵ Pa), preferably from 0.5 mm HgA to 250 mm HgA (66.5 Pa to 0,33.10⁵ Pa). The residence time of the mixture in the evaporator ranges from 0.2 minutes to 10 minutes, preferably about 2 minutes or less, although the time is not critical and may be extended in excess of 10 minutes if so desired.

Thin film evaporators are per se well known in the art. At the exit end of the evaporator, a distillation column is attached. The reaction product contains 4-acetoxystyrene, water, and some unreacted 4-acetoxyphenyl methylcarbinol. The 4-acetoxystyrene and water are collected in the overhead of the distillation column. Tar is collected as a liquid at the exit end of the evaporator. Unreacted methylcarbinol goes back down the distillation column and re-enters the evaporator from the exit end.

The resultant product is 4-acetoxystyrene which is obtained at a selectivity rate of at least from about 70% to about 95%.

The 4-acetoxystyrene monomer may then be polymerized by a free radical initiation process to produce poly(4-acetoxystyrene) such that it has a molecular weight in the range of from 1,000 to 800,000, preferably 5,000 to 500,000 or more preferably 5,000 to 300,000. This intermediate is then hydrolyzed with a base or acid to form poly(4-hydroxystyrene) such that it also has the aforesaid molecular weight range. One preferred free radical initiator is azoisobutyronitrile. Other azo type initiators are also suitable. Still others non-exclusively include peroxides such as benzoyl peroxide, and di-t-butyl peroxide. It is predicted that essentially any free radical initiation system will serve in the same fashion. One preferred hydrolyzing agent is tetramethyl ammonium hydroxide. Other hydrolyzing agents non-exclusively include aqueous NH₃, NaOH, KOH, HCl, and H₂SO₄.

The following non-limiting examples serve to illustrate the invention.

### Example 1

In a glass thin film evaporator, 4-acetoxyphenylmethyl carbinol containing 1% by weight of KHSO₄ and 1% by weight of 4-tert-butyl catechol are fed at a rate of 3.2 g/min. The thin film evaporator is heated to 240^{o}C and the pressure is 242 mm HgA (0,3218.10⁵ Pa). The total feed is 45.1 g and contains 83.3% 4-acetoxy phenylmethyl carbinol by weight. The total overhead collected is 38.1 g. The analysis of the overhead by gas chromatography is 67.5% 4-acetoxystyrene, 11.4% 4-acetoxy phenylmethyl carbinol and the balance is due to other components. The 4-acetoxy phenylmethyl carbinol conversion is 88.4% and the selectivity to 4-acetoxystyrene is 86%.

### Example 2

In a glass thin film evaporator, 4-acetoxy phenylmethyl carbinol containing 1% by weight of KHSO₄ and 1% by weight of 4-tert-butyl catechol are fed at a rate of 3.2 g/min. The thin film evaporator is heated to 220^{o}C and the pressure is 157 mm HgA (0.208.10⁵ Pa). The total feed is 45.6 g and contains 83.3% 4-acetoxy phenylmethyl carbinol by weight. The total overhead collected is 36.5 g. The analysis of the overhead by gas chromatography is 64.1% 4 acetoxystyrene, 15.8% 4-acetoxy phenylmethyl carbinol and the balance is due to other components. The 4-acetoxy phenylmethyl carbinol conversion is 84.9% and the selectivity to 4-acetoxystyrene is 80.7%.

### Example 3

In a glass thin film evaporator, 4-acetoxyphenylmethyl carbinol containing 2% by weight of KHSO₄ and 1% by weight of 4-tert-butyl catechol are fed at a rate of 1.9 g/min. The thin film evaporator is heated to 240^{o}C and the pressure is 155 mm HgA (0,206.10⁵ Pa). The total feed is 250 g and contains 83.3% 4-acetoxy phenylmethyl carbinol by weight. The total overhead collected is 216.2 g. The analysis of the overhead by gas chromatography is 68.0% 4-acetoxystyrene, 8.7% 4-acetoxy phenylmethyl carbinol and the balance is due to other components. The 4-acetoxy phenylmethyl carbinol conversion is 91.0% and the selectivity to 4-acetoxystyrene is 86.3%.

### Example 4

In a glass thin film evaporator, 4-acetoxyphenylmethyl carbinol containing 5% by weight of KHSO₄ and 2% by weight of 4-tert-butyl catechol are fed at a rate of 1.6 g/min. The thin film evaporator is heated to 240^{o}C and the pressure is 155 mm HgA (0,205.10⁵ Pa). The total feed is 400 g and contains 59.7% 4-acetoxy phenylmethyl carbinol by weight. The total overhead collected is 295 g. The analysis of the overhead by gas chromatography is 62.3% 4-acetoxystyrene, 11.4% 4-acetoxy phenylmethyl carbinol and the balance is due to other components. The 4-acetoxy phenylmethyl carbinol conversion is 87.9 and the selectivity to 4-acetoxystyrene is 84.4%.

### Example 5

In a glass thin film evaporator, 4-acetoxyphenylmethyl carbinol containing 1% by weight of KHSO₄ and 1% by weight of 4-tert-butyl catechol are fed at a rate of 11.2 g/min. The thin film evaporator is heated to 240^{o}C and the pressure is 155 mm HgA (0,205.10⁵ Pa). The total feed is 78.1 g and contains 83.3% 4-acetoxy phenylmethyl carbinol by weight. The total overhead collected is 74.1 g. The analysis of the overhead by gas chromatography is 68.7% 4-acetoxystyrene, 9.5% 4-acetoxy phenylmethyl carbinol and the balance is due to other components. The 4-acetoxy phenylmethyl carbinol conversion is 89.1% and the selectivity to 4-acetoxystyrene is 97.5%.

### Example 6

In a glass thin film evaporator, 4-acetoxyphenylmethyl carbinol containing 1% by weight of KHSO₄ and 1% by weight of 4-tert-butyl catechol are fed at a rate of 1.8 g/min. The thin film evaporator is heated to 240^{o}C and the pressure is 155 mm HgA (0,205.10⁵ Pa). The total feed is 55.8 g and contains 83.3% 4-acetoxy phenylmethyl carbinol by weight. The total overhead collected is 46.5 g. The analysis of the overhead by gas chromatography is 68.0% 4-acetoxystyrene, 8.7% 4-acetoxy phenylmethyl carbinol and the balance is due to other components. The 4-acetoxy phenylmethyl carbinol conversion is 91.3% and the selectivity to 4-acetoxystyrene is 82.8%.

## Claims

1. A method for producing 4-acetoxystyrene which comprises heating 4-acetoxyphenyl methylcarbinol, in the presence of a catalytic amount of an acid catalyst, at a temperature of from 85°C to 300°C under a pressure of from 0.1 mm HgA to 760 mm HgA (1,013.10⁵ Pa to 13,3 Pa) in a thin film evaporator for a period of time of from 0.2 minutes to 10 minutes.

2. The method of claim 1 wherein the catalyst is selected from the group consisting of alumina, titania, silica gel and mineral acids.

3. The method of claim 1 wherein the catalyst is KHSO₄.

4. The method of claim 1 wherein the reaction is conducted for a period of from 0.2 minutes to 2 minutes.

5. The method of claim 1 wherein the reaction is conducted at a vacuum of from 0.5 mm HgA to 250 mm HgA (66,5 Pa to 0,33.10⁵ Pa).

6. A process for the production of 4-acetoxystyrene which comprises:
a) acylating phenol with acetic anhydride to produce 4-hydroxyacetophenone; and
b) acylating the 4-hydroxyacetophenone with acetic anhydride to form 4-acetoxyacetophenone; and
c) heating 4-acetoxyacetophenone at a temperature of from 54°C to 120°C in the presence of at least a stoichiometric amount of hydrogen, and a catalytic amount of a catalyst selected from the group consisting of Pd/C or activated nickel in the absence of a solvent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300°C under a pressure of from 0.1 mm HgA to 760 mm HgA (13,3 Pa to 1,013.10⁵ Pa) in a thin film evaporator for a period of time of from 0.2 minutes to 10 minutes.

7. The method of claim 6 wherein the step (d) catalyst is selected from the group consisting of alumina, titania, silica gel and mineral acids.

8. The method of claim 6 wherein the step (d) catalyst is KHSO₄.

9. The method of claim 6 wherein the step (d) reaction is conducted for a period of from 0.2 minutes to 2 minutes.

10. The method of claim 6 wherein the step (d) reaction is conducted at a vacuum of from 0.5 mm HgA to 250 mm HgA (66,5 Pa to 0,33.10⁵ Pa).

11. The method of claim 6 wherein the step (c) catalyst is Pd/C.

12. The method of claim 6 wherein the step (c) catalyst is Raney Nickel.

13. The method of claim 6 wherein the reaction step (c) is conducted until substantial completion of hydrogenation.

14. The method of claim 6 wherein the reaction step (c) is conducted at a pressure of from 14.7 psig to 5,000 psig (2,026.10⁵ Pa to 345,74.10⁵ Pa).

15. A process for the production of poly(4-acetoxystyrene) which comprises:
a) acylating phenol with acetic anhydride to produce 4-hydroxyacetophenone; and
b) acylating the 4-hydroxyacetophenone with acetic anhydride to form 4-acetoxyacetophenone; and
c) heating 4-acetoxyacetophenone at a temperature of from 54°C to 120°C in the presence of at least a stoichiometric amount of hydrogen, and a catalytic amount of a catalyst selected from the group consisting of Pd/C or activated nickel in the absence of a solvent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300°C under a pressure of from 0.1 mm HgA to 760 mm HgA (1,013.10⁵ Pa to 13,3 Pa) in a thin film evaporator for a period of time of from 0.2 minutes to 10 minutes, and
e) free radical polymerizing the 4-acetoxystyrene to form poly(4-acetoxystyrene) having a molecular weight in the range of from 1,000 to 800,000.

16. The method of claim 15 wherein the reaction step (c) is conducted until substantial completion of hydrogenation.

17. The method of claim 15 wherein the reaction step (c) is conducted at a pressure of from 14 psig to 5,000 psig (2,026.10⁵ Pa to 345,74.10⁵ Pa).

18. The method of claim 15 wherein the step (d) catalyst is selected from the group consisting of alumina, titania, silica gel and mineral acids.

19. The method of claim 15 wherein the step (d) catalyst is KHSO₄.

20. The method of claim 15 wherein the step (d) reaction is conducted for a period of from 0.2 minutes to 2 minutes.

21. The method of claim 15 wherein the step (d) reaction is conducted at a vacuum of from 0.5 mm HgA to 250 mm HgA (66,5 Pa to 0,33.10⁵ Pa).

22. A process for the production of poly(4-hydroxystyrene) which comprises:
a) acylating phenol with acetic anhydride to produce 4-hydroxyacetophenone; and
b) acylating the 4-hydroxyacetophenone with acetic anhydride to form 4-acetoxyacetophenone; and
c) heating 4-acetoxyacetophenone at a temperature of from 54°C to 120°C in the presence of at least a stoichiometric amount of hydrogen, and a catalytic amount of a catalyst selected from the group consisting of Pd/C or activated nickel in the absence of a solvent, for a sufficient time to produce 4-acetoxyphenyl methylcarbinol; and
d) heating 4-acetoxyphenyl methylcarbinol, in the presence of a catalytic amount of an acid catalyst, at a temperature of from 8°C to 300°C under a pressure of from 0.1 mm HgA to 760 mm HgA (13,3 Pa to 1,013.10⁵ Pa). in a thin film evaporator for a period of time of from 0.2 minutes to 10 minutes, and
e) free radical polymerizing the 4-acetoxystyrene to form poly(4-acetoxystyrene) having a molecular weight in the range of from 1,000 to 800,000; and
f) hydrolyzing the poly(4-acetoxystyrene) to form poly(4-hydroxystyrene) having a molecular weight in the range of from 1,000 to 500,000.

23. The method of claim 22 wherein the step (d) catalyst is selected from the group consisting of alumina, titania, silica gel and mineral acids.

24. The method of claim 22 wherein the step (d) catalyst is KHSO₄.

25. The method of claim 22 wherein the step (d) reaction is conducted for a period of from 0.2 minutes to 2 minutes.

26. The method of claim 22 wherein the step (d) reaction is conducted at a vacuum of from 0.5 mm HgA to 250 mm HgA (66,5 Pa to 0,33.10⁵ Pa).

27. The method of claim 22 wherein the reaction step (c) is conducted until substantial completion of hydrogenation.

28. The method of claim 22 wherein the reaction step (c) is conducted at a pressure of from 14.7 psig to 5,000 psig (2,026.10⁵ Pa to 345,74.10⁵ Pa).

29. A process for the production of 4-acetoxystyrene which comprises:
a) acylating phenol with acetic anhydride to produce 4-hydroxyacetophenone; and
b) acylating the 4-hydroxyacetophenone with acetic anhydride to form 4-acetoxyacetophenone; and
c) hydrogenating 4-acetoxyacetophenone with a suitable reagent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300°C under a pressure of from 0.1 mm HgA to 760 mm HgA (13,3 Pa to 1,013.10⁵ Pa) in a thin film evaporator for a period of time of from 0.2 minutes to 10 minutes.

30. The method of claim 29 wherein the step (d) catalyst is selected from the group consisting of alumina, titania, silica gel, mineral acids and KHSO₄.

31. The method of claim 29 wherein said step (c) reagent is selected from the group consisting of NaBH₄, lithium aluminum hydride, hydrogen and diisobutyl aluminum hydride.

32. A process for the production of poly(4-acetoxystyrene) which comprises:
a) acylating phenol with acetic anhydride to produce 4-hydroxyacetophenone; and
b) acylating the 4-hydroxyacetophenone with acetic anhydride to form 4-acetoxyacetophenone; and
c) hydrogenating 4-acetoxyacetophenone with a suitable reagent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300°C under a pressure of from 0.1 mm HgA to 760 mm HgA (13,3 Pa to 1,013.10⁵ Pa) in a thin film evaporator for a period of time of from 0.2 minutes to 10 minutes; and
e) free radical polymerizing the 4-acetoxystyrene to form poly(4-acetoxystyrene) having a molecular weight in the range of from 1,000 to 800,000.

33. The method of claim 32 wherein the step (d) catalyst is selected from the group consisting of alumina, titania, silica gel, mineral acids, and KHSO₄.

34. The method of claim 32 wherein said step (c) reagent is selected from the group consisting of NaBH₄, lithium aluminum hydride, hydrogen and diisobutyl aluminum hydride.

35. A process for the production of poly(4-hydroxystyrene) which comprises:
a) acylating phenol with acetic anhydride to produce 4-hydroxacetophenone; and
b) acylating the 4-hydroxyacetophenone with acetic anhydride to form 4-acetoxyacetophenone; and
c) hydrogenating 4-acetoxyacetophenone with a suitable reagent, for a sufficient time to produce 4-acetoxyphenyl methylcarbinol; and
d) heating 4-acetoxyphenyl methylcarbinol, in the presence of a catalytic amount of an acid catalyst, at a temperature of from 85°C to 300 °C under a pressure of from 0.1 mm HgA to 760 mm HgA (13,3 Pa to 1,013.10⁵ Pa) in a thin film evaporator for a period of time of from 0.2 minutes to 10 minutes; and
e) free radical polymerizing the 4-acetoxystyrene to form poly(4-acetoxystyrene) having a molecular weight in the range of from 1,000 to 800,000; and
f) hydrolyzing the poly(4-acetoxystyrene) to form poly(4-hydroxystyrene) having a molecular weight in the range of from 1,000 to 500,000.

36. The method of claim 35 wherein the step (d) catalyst is selected from the group consisting of alumina, titania, silica gel, mineral acids, and KHSO₄.

37. The method of claim 35 wherein said step (c) reagent is selected from the group consisting of NaBH₄, lithium aluminum hydride, hydrogen and diisobutyl aluminum hydride.

## Patentansprüche

1. Verfahren zur Herstellung von 4-Acetoxystyrol, umfassend das Erhitzen von 4-Acetoxyphenylmethylcarbinol in Gegenwart einer katalytischen Menge eines Säure-Katalysators auf eine Temperatur von 85 °C bis 300 °C unter einem Druck von 0,1 mm HgA bis 760 mm HgA (1,013·10⁵ Pa bis 13,3 Pa) in einem Dünnfilm-Verdampfer für die Dauer einer Zeitspanne von 0,2 min bis 10 min.

2. Verfahren nach Anspruch 1, worin der Katalysator aus der aus Aluminiumoxid, Titanoxid, Silicagel und Mineralsäuren bestehenden Gruppe ausgewählt ist.

3. Verfahren nach Anspruch 1, worin der Katalysator KHSO₄ ist.

4. Verfahren nach Anspruch 1, worin die Reaktion während einer Dauer von 0,2 min bis 2 min durchgeführt wird.

5. Verfahren nach Anspruch 1, worin die Reaktion unter einem Vakuum von etwa 0,5 mm HgA bis 250 mm HgA (66,5 Pa bis 0,33·10⁵ Pa) durchgeführt wird.

6. Verfahren zur Herstellung von 4-Acetoxystyrol, umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) das Erhitzen des 4-Acetoxyacetophenons auf eine Temperatur von 54 °C bis 120 °C in Gegenwart wenigstens einer stöchiometrischen Menge Wasserstoff und einer katalytischen Menge eines Katalysators, der aus der aus Pd/C oder aktiviertem Nickel bestehenden Gruppe ausgewählt ist, in Abwesenheit eines Lösungsmittels während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (13,3 Pa bis 1,013·10⁵ Pa) in einem Dünnfilm-Verdampfer für die Dauer einer Zeitspanne von 0,2 min bis 10 min.

7. Verfahren nach Anspruch 6, worin der Katalysator in Schritt (d) aus der aus Aluminiumoxid, Titanoxid, Silicagel und Mineralsäuren bestehenden Gruppe ausgewählt ist.

8. Verfahren nach Anspruch 6, worin der Katalysator in Schritt (d) KHSO₄ ist.

9. Verfahren nach Anspruch 6, worin die Reaktion in Schritt (d) während einer Dauer von 0,2 min bis 2 min durchgeführt wird.

10. Verfahren nach Anspruch 6, worin die Reaktion in Schritt (d) unter einem Vakuum von etwa 0,5 mm HgA bis 250 mm HgA (66,5 Pa bis 0,33·10⁵ Pa) durchgeführt wird.

11. Verfahren nach Anspruch 6, worin der Katalysator in Schritt (c) Pd/C ist.

12. Verfahren nach Anspruch 6, worin der Katalysator in Schritt (c) Raney-Nickel ist.

13. Verfahren nach Anspruch 6, worin die Reaktion in Schritt (c) im wesentlichen bis zur Vollständigkeit der Hydrierung durchgeführt wird.

14. Verfahren nach Anspruch 6, worin die Reaktion in Schritt (c) bei einem Druck von 14,7 psig bis 5 000 psig (2,026·10⁵ Pa bis 345,74·10⁵ Pa) durchgeführt wird.

15. Verfahren zur Herstellung von Poly(4-acetoxystyrol), umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) das Erhitzen des 4-Acetoxyacetophenons auf eine Temperatur von 54 °C bis 120 °C in Gegenwart wenigstens einer stöchiometrischen Menge Wasserstoff und einer katalytischen Menge eines Katalysators, der aus der aus Pd/C oder aktiviertem Nickel bestehenden Gruppe ausgewählt ist, in Abwesenheit eines Lösungsmittels während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (1,013·10⁵ Pa bis 13,3 Pa) in einem Dünnfilm-Verdampfer für die Dauer einer Zeitspanne von 0,2 min bis 10 min; und
e) die radikalische Polymerisation des 4-Acetoxystyrols zur Bildung von Poly(4-acetoxystyrol) mit einem Molekulargewicht im Bereich von 1 000 bis 800 000.

16. Verfahren nach Anspruch 15, worin die Reaktion in Schritt (c) im wesentlichen bis zur Vollständigkeit der Hydrierung durchgeführt wird.

17. Verfahren nach Anspruch 15, worin die Reaktion in Schritt (c) bei einem Druck von 14 psig bis 5 000 psig (2,026·10⁵ Pa bis 345,74·10⁵ Pa) durchgeführt wird.

18. Verfahren nach Anspruch 15, worin der Katalysator in Schritt (d) aus der aus Aluminiumoxid, Titanoxid, Silicagel und Mineralsäuren bestehenden Gruppe ausgewählt ist.

19. Verfahren nach Anspruch 15, worin der Katalysator in Schritt (d) KHSO₄ ist.

20. Verfahren nach Anspruch 15, worin die Reaktion in Schritt (d) während einer Dauer von 0,2 min bis 2 min durchgeführt wird.

21. Verfahren nach Anspruch 15, worin die Reaktion in Schritt (d) unter einem Vakuum von etwa 0,5 mm HgA bis 250 mm HgA (66,5 Pa bis 0,33·10⁵ Pa) durchgeführt wird.

22. Verfahren zur Herstellung von Poly(4-hydroxystyrol), umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) das Erhitzen des 4-Acetoxyacetophenons auf eine Temperatur von 54 °C bis 120 °C in Gegenwart wenigstens einer stöchiometrischen Menge Wasserstoff und einer katalytischen Menge eines Katalysators, der aus der aus Pd/C oder aktiviertem Nickel bestehenden Gruppe ausgewählt ist, in Abwesenheit eines Lösungsmittels während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (13,3 Pa bis 1,013·10⁵ Pa) in einem Dünnfilm-Verdampfer für die Dauer einer Zeitspanne von 0,2 min bis 10 min; und
e) die radikalische Polymerisation des 4-Acetoxystyrols zur Bildung von Poly(4-acetoxystyrol) mit einem Molekulargewicht im Bereich von 1 000 bis 800 000; und
f) die Hydrolyse des Poly(4-acetoxystyrols) zur Bildung eines Poly(4-hydroxystyrols) mit einem Molekulargewicht im Bereich von 1 000 bis 500 000.

23. Verfahren nach Anspruch 22, worin der Katalysator in Schritt (d) aus der aus Aluminiumoxid, Titanoxid, Silicagel und Mineralsäuren bestehenden Gruppe ausgewählt ist.

24. Verfahren nach Anspruch 22, worin der Katalysator in Schritt (d) KHSO₄ ist.

25. Verfahren nach Anspruch 22, worin die Reaktion in Schritt (d) während einer Dauer von 0,2 min bis 2 min durchgeführt wird.

26. Verfahren nach Anspruch 22, worin die Reaktion in Schritt (d) unter einem Vakuum von etwa 0,5 mm HgA bis 250 mm HgA (66,5 Pa bis 0,33·10⁵ Pa) durchgeführt wird.

27. Verfahren nach Anspruch 22, worin die Reaktion in Schritt (c) im wesentlichen bis zur Vollständigkeit der Hydrierung durchgeführt wird.

28. Verfahren nach Anspruch 22, worin die Reaktion in Schritt (c) bei einem Druck von 14,7 psig bis 5 000 psig (2,026·10⁵ Pa bis 345,74·10⁵ Pa) durchgeführt wird.

29. Verfahren zur Herstellung von 4-Acetoxystyrol, umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) die Hydrierung des 4-Acetoxyacetophenons mit einem geeigneten Reagens während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (13,3 Pa bis 1,013·10⁵ Pa) in einem Dünnfilm-Verdampfer für die Dauer einer Zeitspanne von 0,2 min bis 10 min.

30. Verfahren nach Anspruch 29, worin der Katalysator in Schritt (d) aus der aus Aluminiumoxid, Titanoxid, Silicagel, Mineralsäuren und KHSO₄ bestehenden Gruppe ausgewählt ist.

31. Verfahren nach Anspruch 29, worin das Reagens in Schritt (c) aus der aus NaBH₄, Lithiumaluminiumhydrid, Wasserstoff und Diisobutylaluminiumhydrid bestehenden Gruppe ausgewählt ist.

32. Verfahren zur Herstellung von Poly(4-acetoxystyrol), umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) die Hydrierung des 4-Acetoxyacetophenons mit einem geeigneten Reagens während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (13,3 Pa bis 1,013·10⁵ Pa) in einem Dünnfilm-Verdampfer für die Dauer einer Zeitspanne von 0,2 min bis 10 min; und
e) die radikalische Polymerisation des 4-Acetoxystyrols zur Bildung von Poly(4-acetoxystyrol) mit einem Molekulargewicht im Bereich von 1 000 bis 800 000.

33. Verfahren nach Anspruch 32, worin der Katalysator in Schritt (d) aus der aus Aluminiumoxid, Titanoxid, Silicagel, Mineralsäuren und KHSO₄ bestehenden Gruppe ausgewählt ist.

34. Verfahren nach Anspruch 32, worin das Reagens in Schritt (c) aus der aus NaBH₄, Lithiumaluminiumhydrid, Wasserstoff und Diisobutylaluminiumhydrid bestehenden Gruppe ausgewählt ist.

35. Verfahren zur Herstellung von Poly(4-hydroxystyrol), umfassend
a) die Acylierung von Phenol mit Essigsäureanhydrid zur Herstellung von 4-Hydroxyacetophenon; und
b) die Acylierung des 4-Hydroxyacetophenons mit Essigsäureanhydrid zur Herstellung von 4-Acetoxyacetophenon; und
c) die Hydrierung des 4-Acetoxyacetophenons mit einem geeigneten Reagens während einer hinreichend langen Zeit, um 4-Acetoxyphenylmethylcarbinol zu erzeugen; und
d) das Erhitzen des 4-Acetoxyphenylmethylcarbinols in Gegenwart einer katalytischen Menge eines Säure-Katalysators auf eine Temperatur von 85 °C bis 300 °C unter einem Druck von 0,1 mm HgA bis 760 mm HgA (13,3 Pa bis 1,013·10⁵ Pa) in einem Dünnfilm-Verdampfer für die Dauer einer Zeitspanne von 0,2 min bis 10 min; und
e) die radikalische Polymerisation des 4-Acetoxystyrols zur Bildung von Poly(4-acetoxystyrol) mit einem Molekulargewicht im Bereich von 1 000 bis 800 000; und
f) die Hydrolyse des Poly(4-acetoxystyrols) zur Bildung eines Poly(4-hydroxystyrols) mit einem Molekulargewicht im Bereich von 1 000 bis 500 000.

36. Verfahren nach Anspruch 35, worin der Katalysator in Schritt (d) aus der aus Aluminiumoxid, Titanoxid, Silicagel, Mineralsäuren und KHSO₄ bestehenden Gruppe ausgewählt ist.

37. Verfahren nach Anspruch 35, worin das Reagens in Schritt (c) aus der aus NaBH₄, Lithiumaluminiumhydrid, Wasserstoff und Diisobutylaluminiumhydrid bestehenden Gruppe ausgewählt ist.

## Revendications

1. Un procédé de préparation de 4-acétoxystyrène qui consiste à chauffer le 4-acétoxyphénylméthylcarbinol dans un évaporateur à film mince, en présence d'une quantité catalytique d'un catalyseur acide, à une température comprise entre 85 et 300°C, sous une pression comprise entre 1,013.10⁵ Pa et 13,3 Pa (entre 0,1 mm HgA et 760 mm HgA) et pendant une période de temps comprise entre 0,2 et 10 minutes.

2. Le procédé selon la revendication 1, caractérisé en ce que le catalyseur est choisi dans le groupe constitué par gel de silice, alumine, titane et acides minéraux.

3. Le procédé selon la revendication 1, caractérisé en ce que le catalyseur est KHSO₄.

4. Le procédé selon la revendication 1, caractérisé en ce que la réaction est réalisée pendant une période comprise entre 0,2 et 2 minutes.

5. Le procédé selon la revendication 1, caractérisé en ce que la réaction est effectuée sous un vide compris entre 66,5 Pa et 0,33 Pa (entre 0,5 mm HgA et 250 mm HgA).

6. Un procédé de préparation de 4-acétoxystyrène qui comprend les étapes suivantes:
a) acylation du phénol par l'anhydride acétique pour obtenir la 4-hydroxyacétophénone;
b) acylation de la 4-hydroxyacétophénone avec de l'anhydride acétique pour former la 4-acétoxyacétophénone;
c) chauffage de la 4-acétoxyacétophénone à une température comprise entre 54 et 120°C, en présence d'une quantité au moins stoechiométrique d'hydrogène et d'une quantité catalytique d'un catalyseur choisi dans le groupe constitué par Pd/C ou par du nickel activé, en l'absence de solvant et pendant un temps suffisant pour obtenir le 4-acétoxyphénylméthylcarbinol; et
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 13,3 et 1,013.10⁵ Pa (0,1 mm HgA et 760 mm HgA) et pendant une période de temps comprise entre 0,2 et 10 minutes.

7. Le procédé selon la revendication 6, caractérisé en ce que le catalyseur de l'étape (d) est choisi dans le groupe constitué par gel de silice, alumine, titane et acides minéraux.

8. Le procédé selon la revendication 6, caractérisé en ce que le catalyseur de l'étape (d) est KHSO₄.

9. Le procédé selon la revendication 6, caractérisé en ce que la réaction de l'étape (d) est réalisée pendant une période comprise entre 0,2 et 2 minutes.

10. Le procédé selon la revendication 6, caractérisé en ce que la réaction de l'étape (d) est réalisée sous un vide compris entre 66,5 et 0,33.10⁵ Pa (entre 0,5 mm HgA et 250 mm HgA).

11. Le procédé selon la revendication 6, caractérisé en ce que le catalyseur de l'étape (c) est Pd/C.

12. Le procédé selon la revendication 6, caractérisé en ce que le catalyseur de l'étape (c) est le nickel de Raney.

13. Le procédé selon la revendication 6, caractérisé en ce que la réaction de l'étape (c) est réalisée sensiblement jusqu'à l'achèvement de l'hydrogénation.

14. Le procédé selon la revendication 6, caractérisé en ce que la réaction de l'étape (c) est réalisée à une pression comprise entre 2,026.10⁵ Pa et 345,74.10⁵ Pa (entre 14,7 psig et 5000 psig).

15. Un procédé de préparation de poly(4-acétoxystyrène) qui comprend les étapes suivantes:
a) acylation du phénol par l'anhydride acétique pour obtenir la 4-hydroxyacétophénone;
b) acylation de la 4-hydroxyacétophénone par l'anhydride acétique pour former la 4-acétoxyacétophénone;
c) chauffage de la 4-acétoxyphénone à une température comprise entre 54 et 120°, en présence d'une quantité au moins stoechiométrique d'hydrogène et d'une quantité catalytique d'un catalyseur choisi dans le groupe constitué par Pd/C ou par du nickel activé, en l'absence de solvant et pendant une durée suffisante pour obtenir le 4-acétoxyphénylméthylcarbinol;
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 1,013.10⁵ Pa et 13,3 Pa (entre 0,1 mm HgA et 760 mm HgA) et pendant une période de temps comprise entre 0,2 et 10 minutes; et
e) polymérisation en présence de radicaux libres du 4-acétoxystyrène pour obtenir un poly(4-acétoxystyrène) dont le poids moléculaire est compris entre 1000 et 800 000.

16. Le procédé selon la revendication 15, caractérisé en ce que l'étape (d) est réalisée sensiblement jusqu'à achèvement de l'hydrogénation.

17. Le procédé selon la revendication 15, caractérisé en ce que la réaction de l'étape (c) est réalisée à une pression comprise entre 2,026.10⁵ Pa et 345,74.10⁵ Pa (entre 14 psig et 5000 psig).

18. Le procédé selon la revendication 15, caractérisé en ce que le catalyseur de l'étape (d) est choisi dans le groupe constitué par gel de silice, alumine, titane et acides minéraux.

19. Le procédé selon la revendication 15, caractérisé en ce que le catalyseur de l'étape (d) est KHSO₄.

20. Le procédé selon la revendication 15, caractérisé en ce que la réaction de l'étape (d) est réalisée pendant une période comprise entre 0,2 et 2 minutes.

21. Le procédé selon la revendication 15, caractérisé en ce que la réaction de l'étape (d) est réalisée sous un vide compris entre 66,5 Pa et 0,33.10⁵ Pa (entre 0,5 mm HgA et 250 mm HgA).

22. Un procédé de préparation de poly(4-hydroxystyrène) qui comprend les étapes suivantes:
a) acylation du phénol par l'anhydride acétique pour obtenir la 4-hydroxyacétophénone;
b) acylation de la 4-hydroxyacétophénone par l'anhydride acétique pour former la 4-acétoxyacétophénone;
c) chauffage de la 4-acétoxyphénone à une température comprise entre 54 et 120°C, en présence d'une quantité au moins stoechiométrique d'hydrogène et d'une quantité catalytique d'un catalyseur choisi dans le groupe constitué par Pd/C ou par du nickel activé, en l'absence de solvant et pendant une durée suffisante pour obtenir le 4-acétoxyphénylméthylcarbinol;
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 1,013.10⁵ Pa et 13,3 Pa (entre 0,1 mm HgA et 760 mm HgA) et pendant une période de temps comprise entre 0,2 et 10 minutes;
e) polymérisation en présence de radicaux libres du 4-acétoxystyrène pour obtenir un poly(4-acétoxystyrène) dont le poids moléculaire est compris entre 1000 et 800 000; et
f) hydrolyse du poly(4-acétotoxystyrène) pour former un poly(4-hydroxystyrène) dont le poids moléculaire est compris entre 1000 et 500 000.

23. Le procédé selon la revendication 22, caractérisé en ce que le catalyseur de l'étape (d) est choisi dans le groupe constitué par gel de silice, alumine, titane et acides minéraux.

24. Le procédé selon la revendication 22, caractérisé en ce que le catalyseur de l'étape (d) est KHSO₄.

25. Le procédé selon la revendication 22, caractérisé en ce que la réaction de l'étape (d) est réalisée pendant une période comprise entre 0,2 et 2 minutes.

26. Le procédé selon la revendication 22, caractérisé en ce que la réaction de l'étape (d) est réalisée sous un vide compris entre 66,5 Pa et 0,33.10⁵ Pa (entre 0,5 mm HgA et 250 mm HgA).

27. Le procédé selon la revendication 22, caractérisé en ce que la réaction de l'étape (c) est réalisée sensiblement jusqu'à l'achèvement de l'hydrogénation.

28. Le procédé selon la revendication 22, caractérisé en ce que la réaction de l'étape (c) est réalisée à une pression comprise entre 2,026.10⁵ Pa et 345,74.10⁵ Pa (entre 14,7 psig et 5000 psig).

29. Un procédé de préparation de 4-acétoxystyrène qui comprend les étapes suivantes:
a) acylation du phénol par l'anhydride acétique pour obtenir la 4-hydroxyacétophénone;
b) acylation de la 4-hydroxyacétophénone par l'anhydride acétique pour former la 4-acétoxyacétophénone;
c) hydrogénation de la 4-acétoxyacétophénone par un réactif convenable pendant un temps suffisant pour former le 4-acétoxyphénylméthylcarbinol; et
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 13,3 Pa et 1,013.10⁵ Pa (entre 0,1 mm HgA et 760 mm HgA) et pendant une période de temps comprise entre 0,2 et 10 minutes.

30. Le procédé selon la revendication 29, caractérisé en ce que le catalyseur de l'étape (d) est choisi dans le groupe constitué par gel de silice, alumine, titane, acides minéraux et KHSO₄.

31. Le procédé selon la revendication 29, caractérisé en ce que la réactif de départ dans l'étape (c) est choisi dans le groupe constitué par hydrure d'aluminium, lithium, NaBH₄, hydrure de diisobutylaluminium et hydrure d'hydrogénoaluminium.

32. Un procédé de préparation de poly(4-acétoxystyrène) qui comprend les étapes suivantes:
a) acylation du phénol par l'anhydride acétique pour obtenir la 4-hydroxyacétophénone;
b) acylation de la 4-hydroxyacétophénone par l'anhydride acétique pour former la 4-acétoxyacétophénone;
c) hydrogénation de la 4-acétoxyacétophénone par un réactif convenable pendant un temps suffisant pour former le 4-acétoxyphénylméthylcarbinol;
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 13,3 Pa et 1,013.10⁵ Pa (entre 0,1 mm HgA et 760 mm HgA) et pendant une période de temps comprise entre 0,2 et 10 minutes; et
e) polymérisation en présence de radicaux libres du 4-acétoxystyrène pour obtenir un poly(4-acétoxystyrène) dont le poids moléculaire est compris entre 1000 et 800 000.

33. Le procédé selon la revendication 32, caractérisé en ce que le catalyseur de l'étape (d) est choisi dans le groupe constitué par gel de silice, alumine, titane, acides minéraux et KHSO₄.

34. Le procédé selon la revendication 32, caractérisé en ce que la réactif de départ dans l'étape (c) est choisi dans le groupe constitué par hydrure d'aluminium, lithium, NaBH₄, hydrure de diisobutylaluminium et hydrure d'hydrogénoaluminium.

35. Un procédé de préparation de poly(4-hydroxystyrène) qui comprend les étapes suivantes:
a) acylation du phénol par l'anhydride acétique pour obtenir la 4-hydroxyacétophénone;
b) acylation de la 4-hydroxyacétophénone par l'anhydride acétique pour former la 4-acétoxyacétophénone;
c) hydrogénation de la 4-acétoxyacétophénone par un réactif convenable, pendant un temps suffisant pour former le 4-acétoxyphénylméthylcarbinol;
d) chauffage du 4-acétoxyphénylméthylcarbinol dans un évaporateur à film mince, en présence d'une quantité catalytique d'un catalyseur acide, à une température comprise entre 85 et 300°C, sous une pression comprise entre 13,3 Pa et 1,013.10⁵ Pa (entre 0,1 mm HgA et 760 mm HgA) et pendant une période de temps comprise entre 0,2 et 10 minutes;
e) polymérisation en présence de radicaux libres du 4-acétoxystyrène pour obtenir un poly(4-acétoxystyrène) dont le poids moléculaire est compris entre 1000 et 800 000; et
f) hydrolyse du poly(4-acétotoxystyrène) pour former un poly(4-hydroxystyrène) dont le poids moléculaire est compris entre 1000 et 500 000.

36. Le procédé selon la revendication 35, caractérisé en ce que le catalyseur de l'étape (d) est choisi dans le groupe constitué par gel de silice, alumine, titane, acides minéraux et KHSO₄.

37. Le procédé selon la revendication 35, caractérisé en ce que la réactif de départ dans l'étape (c) est choisi dans le groupe constitué par hydrure d'aluminium, lithium, NaBH₄, hydrure de diisobutylaluminium et hydrure d'hydrogénoaluminium.
